(19) Europäisches Patentamt
European Patent Office
Office européen des brevets

(11) **EP 3 689 901 A1**

(12) **EUROPEAN PATENT APPLICATION**
published in accordance with Art. 153(4) EPC

(43) Date of publication:
**05.08.2020 Bulletin 2020/32**

(21) Application number: **18862547.9**

(22) Date of filing: **09.07.2018**

(51) Int Cl.:
*C07K 14/78* (2006.01)    *A61L 27/02* (2006.01)
*A61L 27/24* (2006.01)    *A61L 27/60* (2006.01)

(86) International application number:
**PCT/JP2018/025846**

(87) International publication number:
**WO 2019/064807 (04.04.2019 Gazette 2019/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **29.09.2017 JP 2017189789**

(71) Applicant: **NATIONAL AGRICULTURE AND FOOD RESEARCH ORGANIZATION**
**Tsukuba-shi, Ibaraki 305-8517 (JP)**

(72) Inventors:
• **NISHIMURA, Ayako**
  **Kashima-shi**
  **Saga 849-1393 (JP)**
• **MIYAUCHI, Shohei**
  **Kashima-shi**
  **Saga 849-1393 (JP)**
• **TAKEZAWA, Toshiaki**
  **Tsukuba-shi**
  **Ibaraki 305-8602 (JP)**
• **MIYAZAKI, Ayumi**
  **Tsukuba-shi**
  **Ibaraki 305-8602 (JP)**

(74) Representative: **Blodig, Wolfgang**
  **Wächtershäuser & Hartz**
  **Patentanwaltspartnerschaft mbB**
  **Weinstrasse 8**
  **80333 München (DE)**

(54) **METHOD FOR PRODUCING COLLAGEN VITRIGEL, METHOD FOR PRODUCING PURIFIED COLLAGEN VITRIGEL, AND COLLAGEN VITRIGEL AND PURIFIED COLLAGEN VITRIGEL PRODUCED BY SAID METHODS**

(57)    An object is to produce a collagen vitrigel, which has a high film strength even without performing a crosslinking treatment, and is easily produced and processed industrially and mechanically, and is also easy to handle and is highly safe, from collagen, and a purified product thereof. In order to achieve the object, it is directed to a method for producing a collagen vitrigel characterized by gelling collagen with a gelling agent containing an inorganic carbonate and a compound selected from the group consisting of an inorganic chloride and an inorganic phosphate, subsequently vitrifying the obtained collagen gel, and further subjecting the vitrified collagen gel to a hydration treatment; a method for producing a purified collagen vitrigel characterized by desalting, equilibrating, and further drying the collagen vitrigel; and a collagen vitrigel and a purified collagen vitrigel obtained by these methods.

[FIG.1]

## Description

Technical Field

The present invention relates to methods for producing a collagen vitrigel and a purified product thereof. More particularly, it relates to a collagen vitrigel that is obtained by fibril formation of collagen and can be utilized for medical use or the like, a purified collagen vitrigel, and production methods therefor.

Background Art

Collagen is a protein present in the living body. Collagen makes up about 30% of the whole-body protein content in humans, and is particularly abundant in a skin, a bone, a cartilage, a tendon, and a blood vessel wall. Collagen has a molecular weight of about 300,000, and a triple helical structure composed of three polypeptide chains having a molecular weight of about 100,000 is formed. In the collagen, there exist many molecular species having a different amino acid sequence order and a different amino acid composition ratio depending on an animal from which the collagen is derived and a tissue thereof.

It is known that collagen plays a role of a scaffold for cells as an extracellular matrix in vivo, and also has an effect on proliferation, differentiation, and morphogenesis, and collagen has been utilized as a cell culture carrier for a long time, and has also been applied as a biological graft material recently.

As the cell culture carrier among these, various collagen-coated culture dishes and flasks are commercially available, and also an embedded culture method in which cells are dispersed and cultured in a collagen gel is known. On the other hand, as the biological graft material, there exist a collagen gel material having cells carried thereon, an injectable gel that is grafted in a solution state and gelled in vivo, a material processed into a film shape or a sponge shape by drying a collagen gel, and the like.

Further, as the biological graft material composed of collagen, NPL 1 discloses a cartilage graft material, PTL 1 discloses a gelling material for in vivo injection, and PTL 2 discloses an artificial skin material, and the like. In such a manner, the biological graft material composed of collagen is various in shape, but there are lots of materials processed and shaped by applying a collagen gel.

It is known that such a collagen gel can be obtained by placing a collagen solution under physiological salt concentration, hydrogen-ion concentration, and temperature conditions, and therefore, as a gelling agent, various cell culture solutions, physiological saline, a buffer solution having a buffer capacity in a neutral region is generally used.

Incidentally, there is a "collagen vitrigel" as a novel collagen material expected to be applied as a biological graft material. The "vitrigel (Registered Trademark No. 5602094)" is a novel academic term named by Takezawa et al. and is defined as a gel in a stable state obtained by rehydration after vitrification of a conventional hydrogel such as an extracellular matrix (NPL 2). The collagen vitrigel formed from collagen that is an extracellular matrix is composed of high-density collagen fibrils.

A thin film utilizing the collagen vitrigel has characteristics that it is thinner than a conventional plate-shaped collagen gel material and has a high strength, and its application as a biological graft material is expected, and for example, NPL 3 discloses a cartilage graft material composed of a collagen vitrigel thin film obtained using porcine skin-derived atelocollagen as a raw material. Further, a collagen vitrigel dried body thin film obtained using bovine skin-derived native collagen as a raw material has already been commercialized as a substratum for cell culture (Kanto Chemical Co., Inc., # ad-MED vitrigel (trade name)).

In the production of the collagen vitrigel, first, it is necessary to prepare a collagen gel. Conventionally, in the preparation of a collagen gel, as described above, a general cell culture solution, for example, Dulbecco's modified Eagle's medium (DMEM) is used as a gelling agent. Further, NPL 4 discloses a method for preparing a collagen vitrigel from porcine skin-derived atelocollagen using DMEM supplemented with HEPES (4-(2-hydroxyethyl)-1-piperazineethanesulfonic acid) as a gelling agent.

However, a method for obtaining a collagen gel using DMEM as a gelling agent is a preparation method that has been conventionally used in an embedded culture method in an in vitro test, but is not preferred as a method for preparing a biological graft material. The reason is that in DMEM, more than 30 types of compounds are contained, however, only a few compounds among them are involved in the gelling of collagen, and in order to use DMEM for preparing a collagen vitrigel as a biological graft material, it is necessary to confirm the safety of more than 30 types of compounds and to set a residual tolerance limit thereof so as to impose a heavy burden on a manufacturer.

As one example of a method for solving the above problem, the above NPL 4 discloses a collagen vitrigel obtained using a phosphate buffered saline (PBS) supplemented with HEPES as a gelling agent. However, the collagen vitrigel prepared using the solution as the gelling agent has a problem that the strength is low and it is not suitable as a biological graft material, and also it has been pointed out that there is also a safety concern about HEPES itself.

Further, the collagen vitrigel is not limited to one obtained by the above-mentioned method, and as a method for compensating the low strength of the collagen vitrigel, a method for increasing the film strength through chemical crosslinking by addition of a crosslinking agent or through physical crosslinking by irradiation with a UV ray or a gamma ray is known. However, when it is assumed to be used as a biological graft material, there is a concern about the biotoxicity of the added compound in the crosslinking with the compound, and also there is a concern about the denaturation of collagen by the irradiation with a UV ray or a gamma ray, and therefore, it is desired not to perform such a crosslinking treatment.

In view of the above, the development of a method capable of preparing a collagen vitrigel having a sufficient strength in the end by selecting a highly safe compound as a component of a gelling agent and while simplifying the composition as much as possible has been demanded.

Citation List

Patent Literature

PTL 1: Japanese Patent No. 6071468
PTL 2: Japanese Patent No. 4674211

Non Patent Literature

NPL 1: Katsura Sugawara, "Recent Advancement in the Treatment of Cartilage Defects by Cultured Cartilage", Journal of Artificial Organs, The Official Journal of the Japanese Society for Artificial Organs, 2013, Vol. 42, No. 3, pp. 198-200
NPL 2: Toshiaki Takezawa, "Collagen Vitrigel: A Novel Scaffold That Can Facilitate a Three-Dimensional Caluture for Reconstructing Organoids", Cell Transplantaion, (2004), Vol. 13, pp. 463-473
NPL 3: Hideaki Maruki, "Effects of a cell-free method using collagen vitrigel incorporating TGF-$\beta$1 on articular cartilage repair in a rabbit osteochondral defect model", Journal of Biomedical Materials Research B, (2016)
NPL 4: "Research for agri-health translational research project, Research Findings, vol. 561", Ministry of Agriculture, Forestry and Fisheries Agriculture, Forestry and Fisheries Research Council Secretariat, 2016, pp. 315-322

Summary of Invention

Technical Problem

In view of the above circumstances, a task of the present invention is to develop a gelling agent that is composed of only a highly safe compound for the living body and has a simple composition, and to produce a collagen vitrigel, which has a high film strength even without performing a crosslinking treatment, and is easily produced and processed industrially and mechanically, and is easy to handle, from collagen, and a purified product thereof.

That is, an object of the present invention is to provide a collagen vitrigel obtained by fibril formation of collagen using a gelling agent that is composed of only a highly safe compound for the living body and has a simple composition, a purified product thereof, and production methods therefor, and the collagen vitrigel and the purified product thereof having a high film strength, being easily produced and processed industrially and mechanically, being easy to handle, being biologically highly safe, and being usable also for medical purposes, and the production methods therefor at low cost.

Solution to Problem

The present inventors made intensive studies for a gelling agent to be used in the production of a collagen gel, and as a result, they found that a collagen gel can be produced even without using a conventional medium component or organic buffer component by using a solution containing at least one compound of inorganic carbonates such as an inorganic carbonate and an inorganic bicarbonate, and at least one compound of an inorganic chloride and an inorganic phosphate as a gelling agent, and moreover, the obtained collagen gel does not contain a component other than the above-mentioned inorganic components, and therefore is highly safe. Moreover, they found that a collagen vitrigel derived from the collagen gel and a purified product thereof have a high film strength, are easily produced and processed, and are easy to handle, and thus completed the present invention.

That is, the present invention is directed to a method for producing a collagen vitrigel characterized by gelling collagen with a gelling agent containing an inorganic carbonate and a compound selected from the group consisting of an inorganic chloride and an inorganic phosphate, subsequently vitrifying the obtained collagen gel by drying, and further subjecting the vitrified collagen gel to a hydration treatment.

Further, the present invention is directed to a method for producing a purified collagen vitrigel (hydrated body) characterized by desalting and equilibrating a collagen vitrigel prepared by the above-mentioned production method, and a purified collagen vitrigel (dried body) characterized by further drying and revitrifying the purified collagen vitrigel (hydrated body).

Still further, the present invention is directed to a collagen vitrigel and a purified collagen vitrigel obtained by the above-mentioned methods.

Advantageous Effects of Invention

According to the method of the present invention, a medium component or an organic compound such as an organic buffer component is not blended in a gelling agent for collagen, and only an inorganic salt is used, and therefore, a collagen gel in which the amount of a residual component in a produced gel is small, and also its composition is clear, and a problem caused by a contaminant is less likely to occur is obtained.

Accordingly, a collagen vitrigel produced from the collagen gel and further a purified collagen vitrigel derived therefrom are highly safe for the living body, and for example, can be advantageously used for medical applications including biological graft.

Brief Description of Drawings

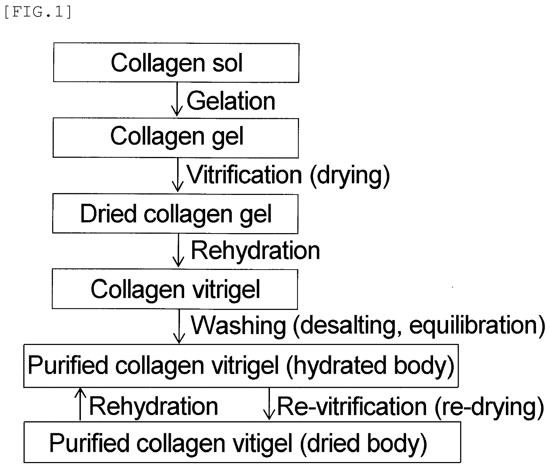

[Fig. 1] Fig. 1 is a view simply showing a production method for a collagen vitrigel and a purified collagen vitrigel of the present invention, a relationship thereof, etc.

Description of Embodiments

In the present description, the collagen vitrigel means a material obtained by rehydrating a collagen gel dried body (collagen xerogel) resulting from vitrification of a collagen gel by drying as shown in Fig. 1. Further, the purified collagen vitrigel means a material obtained by equilibrating the collagen vitrigel after a desalting treatment, a material obtained by revitrification by further drying the equilibrated collagen vitrigel, and a rehydrated material thereof.

The collagen vitrigel of the present invention is obtained by mixing collagen with an aqueous solution containing an inorganic carbonate and a compound (inorganic salt) selected from the group consisting of an inorganic chloride and an inorganic phosphate, thereby preparing a collagen gel, subsequently vitrifying the obtained collagen gel by drying, thereby obtaining a collagen gel dried body, and further hydrating the collagen gel dried body.

More specifically, the collagen vitrigel is produced in the following manner. That is, first, a collagen solution and an aqueous solution containing the above-mentioned inorganic carbonate and a compound selected from the group consisting of an inorganic chloride and an inorganic phosphate (hereinafter sometimes abbreviated as "gelling agent"), thereby forming a collagen sol, and the collagen sol is heated to cause fibril formation, whereby a collagen gel is formed. Subsequently, a collagen gel dried body obtained by drying and vitrifying the collagen gel is subjected to a hydration treatment, whereby a collagen vitrigel is obtained.

Further, the purified collagen vitrigel is obtained by further desalting (washing) and equilibrating a collagen vitrigel obtained as described above, and further drying and revitrifying the equilibrated collagen vitrigel, followed by rehydration as needed.

In order to prepare a collagen vitrigel by the method of the present invention, first, it is necessary to prepare a collagen gel by mixing collagen with a gelling agent.

The collagen to be used in the present invention is not particularly limited with respect to an animal species from which it is derived, and various species can be used. For example, as its origin, mammalian-derived collagen (for example, bovine-derived collagen, porcine-derived collagen, goat-derived collagen, sheep-derived collagen, or monkey-derived collagen), avian-derived collagen (for example, chicken-derived collagen, goose-derived collagen, duck-derived collagen, or ostrich-derived collagen), fish-derived collagen (for example, salmon-derived collagen, sea bream-derived collagen, tuna-derived collagen, tilapia-derived collagen, or shark-derived collagen), reptil-derived collagen (for example, crocodile-derived collagen), amphibian-derived collagen (for example, frog-derived collagen), or invertebrate-derived collagen (for example, jellyfish-derived collagen) can be used. Further, a site from which the collagen is derived is not particularly limited, and for example, a skin, a bone, a cartilage, a muscle, or a scale can be exemplified.

In the present invention, the collagen to be preferably used is collagen that is stable without denaturation at a temperature not higher than 37°C that is the human body temperature. The denaturation temperature of collagen is associated with the habitat of an organism from which it is derived, and collagen of an aquatic organism such as a fish has a denaturation temperature in a lower temperature range than that of a human. Therefore, collagen derived from a terrestrial organism having a denaturation temperature close to that of a human is preferred, and it is preferred to obtain collagen from a livestock animal from the viewpoint of industrial stable supply. Examples of the livestock animal include cattle and swine, however, there is a risk that cattle may have a pathogen of BSE (bovine spongiform encephalopathy) or the like, and therefore, cattle is not preferred, and swine is preferred.

Further, the collagen to be used in the present invention is not limited also with respect to its molecular structure as long as it is fibrous collagen, and examples of a molecular species (type) include type I collagen, type II collagen, type III collagen, and type V collagen. In particular, collagen constituted by type I collagen or type III collagen as a main component is produced in an industrially high yield and can be supplied relatively inexpensively and stably, and thus is preferred. Further, a non-helical structure region (telopeptide) present at the end of a collagen molecule has antigenicity, and therefore, it is more preferred to use atelocollagen obtained by removing the telopeptide by an enzymatic treatment (conversion into atelocollagen).

The collagen is preferably used in a state of a solution in which the collagen is dissolved in a solvent such as water at the time of gelling, and is more preferably an acid-soluble collagen solution at a pH of 2.0 to 6.0. When the pH is lower than 2.0, a collagen molecule may be hydrolyzed, and when the pH is higher than 6.0, collagen may not be sufficiently solubilized, and therefore both pH ranges are not preferred.

Further, the collagen concentration in the collagen solution at the time of gelling is not limited as long as the solubility of collagen and the viscosity of the collagen solution do not hinder the production of the collagen gel, and the physical properties of the obtained collagen vitrigel are sufficient for its application, however, the final concentration after mixing with the gelling agent is preferably within a range of 0.05 w/v% to 5.0 w/v%, and more preferably within a range of 0.2 w/v% to 2.0 w/v%.

On the other hand, the gelling agent to be used in the gelling of collagen is an aqueous solution containing an inorganic carbonate (an inorganic carbonate or an inorganic bicarbonate) and a compound selected from the group consisting of an inorganic chloride and an inorganic phosphate (hereinafter sometimes abbreviated as "inorganic salt") as described above.

In the present invention, the inorganic carbonate to be used as a component of the gelling agent is not limited with respect to its molecular structure as long as it is easily soluble in water, and for example, an inorganic carbonate such as sodium carbonate or potassium carbonate or an inorganic bicarbonate such as sodium bicarbonate or potassium bicarbonate can be used.

Further, as the inorganic salt to be used as another component of the gelling agent is not limited with respect to its molecular structure as long as it belongs to an inorganic chloride or an inorganic phosphate, and is easily soluble in water, and for example, an inorganic chloride such as sodium chloride, potassium chloride, magnesium chloride, or calcium chloride, or an inorganic phosphate such as sodium phosphate, potassium phosphate, disodium hydrogen phosphate, dipotassium hydrogen phosphate, sodium dihydrogen phosphate, potassium dihydrogen phosphate, or calcium dihydrogen phosphate can be used.

The types of the inorganic carbonate and the inorganic salt (the inorganic chloride or the inorganic phosphate) and the combination thereof in the gelling agent are not particularly limited as long as fibril formation (self-assembly) of collagen can be caused when mixing the collagen solution therewith, however, it is necessary to contain at least one inorganic carbonate and at least one compound of an inorganic chloride and an inorganic phosphate.

Further, the pH at 25°C of the solution (collagen sol) after mixing the gelling agent and the collagen solution is preferably within a range of 6.2 to 9.8, and more preferably within a range of 6.8 to 9.0. In addition, as the concentrations of the inorganic carbonate and the inorganic salt in the gelling agent, the ionic strength in the collagen sol is preferably within a range of 0.07 to 0.22, and more preferably within a range of 0.07 to 0.18.

In the present invention, the collagen sol prepared by mixing the gelling agent and the collagen solution is heated to cause fibril formation of collagen, whereby a collagen gel is formed, however, the temperature at that time is preferably determined based on the denaturation temperature of the collagen to be used. The fibril formation of collagen is caused at a temperature in the vicinity of the denaturation temperature of the collagen, and fibril formation is not caused at a temperature much lower than the denaturation temperature. That is, the temperature is preferably within a range that is -20°C or higher with respect to the denaturation temperature and the denaturation temperature or lower. For example, the denaturation temperature of porcine-derived collagen is 41°C, and therefore, the temperature is preferably within a range of 21°C to 41°C.

The collagen gel obtained as described above is subsequently subjected to drying and vitrification, thereby forming a collagen gel dried body (collagen xerogel). The "vitrification" means, for example, a phenomenon in which a hydrogel of a thermally denatured protein such as a hen egg protein (egg white) is dried to sufficiently remove water, thereby converting it into a glass-like material that is hard and has high transparency (Takushi Eisei, "Edible eyeballs from fish", Nature, 1990, Vol. 345, pp. 298-299).

In the method of the present invention, as a drying method for vitrification of the collagen gel, it is preferred to use air drying, and further, the temperature in the air drying is preferably the denaturation temperature of the collagen or lower.

More specifically, preferably, in the air drying, a constant temperature and humidity machine is used, and for example, it is preferred to perform the vitrification of the collagen gel by leaving the collagen gel to stand under the temperature and humidity conditions of about 10°C and 40% RH for 2 days.

The thus obtained collagen gel dried body is subsequently rehydrated, whereby a collagen vitrigel can be formed. As a solution to be used for the rehydration of the collagen gel dried body, an aqueous solution in a neutral pH range (for example, about pH 6.0 to 8.0) such as physiological saline, a phosphate buffer solution, or purified water containing the components to be contained in the gelling agent of the present invention can be exemplified. The solution to be used for the rehydration does not need to have the same composition and concentration as those of the gelling agent when producing the collagen gel, and is not limited thereto.

In the rehydration, it is preferred to hydrate the collagen gel dried body by immersing it in the above D-PBS(-) in an amount of 1 mL or more per 10 mg of the collagen gel dried body for 30 minutes or more. The temperature of the aqueous solution to be used for the hydration is preferably a temperature much lower than the denaturation temperature of the collagen to be used, and is preferably -20°C or lower with respect to the denaturation temperature.

The collagen vitrigel as a hydrated material is prepared as described above. The collagen vitrigel has a high strength itself, and is easily produced and processed industrially and mechanically, and is easy to handle. Then, in the collagen vitrigel, only inorganic compounds such as an inorganic carbonate, an inorganic chloride, or an inorganic phosphate are used as a component of a gelling agent or a rehydrating agent to be used in the production step, and medium components or organic materials that are conventionally used are not used, and therefore, the collagen vitrigel can be used, for example, as a biologically highly safe collagen vitrigel for medical purposes.

Note that by further subjecting the collagen vitrigel to a desalting treatment and an equilibration treatment, followed by drying, a purified collagen vitrigel (dried body) can be formed.

The above-mentioned collagen gel dried body (collagen xerogel) is obtained by drying the collagen gel, and therefore, the inorganic compounds contained in the used gelling agent is concentrated by drying and is apt to be deposited as crystals in some cases. The crystals are unevenly deposited on the surface of the collagen vitrigel dried body, and therefore, the appearance is deteriorated, and also a problem in uniformity of products may occur.

On the other hand, by subjecting the collagen vitrigel to a desalting treatment and an equilibration treatment, a purified collagen vitrigel (hydrated body) having high uniformity and good appearance can be obtained. The desalting treatment performed here is carried out by immersing the collagen vitrigel in an aqueous solution in a neutral pH range (for example, about pH 6.0 to 8.0), but is preferably carried out by immersing it in D-PBS(-). Specifically, for example, the collagen gel dried body is hydrated by immersing it in D-PBS(-) in an amount of 1 mL or more per 10 mg of the collagen gel dried body, thereby forming a collagen vitrigel, and thereafter, D-PBS(-) around the collagen vitrigel is removed, and further the collagen vitrigel is immersed in D-PBS(-) twice of more, whereby desalting of the collagen vitrigel and equilibration thereof with D-PBS(-) can be carried out.

The temperature of the aqueous solution in the operation is preferably a temperature much lower than the denaturation temperature of the collagen to be used, and is preferably -20°C or lower with respect to the denaturation temperature.

Further, in the drying for revitrification of the purified collagen vitrigel (hydrated body), it is preferred to use air drying, and further, the temperature in the air drying is preferably the denaturation temperature of the collagen or lower. More specifically, it is preferred to use a constant temperature and humidity machine in the air drying, and for example, it is preferred to perform the drying by leaving the purified collagen vitrigel (hydrated body) to stand in a constant temperature and humidity machine under the temperature and humidity conditions of 10°C and 40% RH for 1 day.

The collagen vitrigel and the purified collagen vitrigel prepared by the method described above have a high strength per se, and are highly safe as described above, and therefore, can be used particularly as a device for medical purposes such as a cell carrier for regenerative medicine, a wound dressing, an artificial skin, or an adhesion preventive material. Further, as for its shape, it can be processed into an arbitrary shape according to its application, for example, a plate shape, a film shape, a rod shape, a thread shape, a cylindrical shape, a tubular shape, a bag shape, or the like.

Examples

Hereinafter, the present invention will be further specifically described by showing Examples, however, the present invention is not limited to the Examples, and various changes can be made within the scope being not deviated from the technical idea of the present invention.

Example 1

(Preparation of Collagen Solution)

2 g of porcine skin-derived atelocollagen (NH Foods Ltd. # NMP collagen PS) was dissolved in 200 mL of sterile water, whereby a 1 w/v% collagen solution was prepared.

(Preparation of Gelling Agent)

To 500 mL of phosphate-buffered saline (D-PBS(-), Wako Pure Chemical Industries, Ltd. # 045-29795), 1.85 g of sodium bicarbonate (Wako Pure Chemical Industries, Ltd. # 191-01305) was added and dissolved therein, whereby a gelling agent was prepared.

(Preparation of Collagen Vitrigel and Purified Collagen Vitrigel)

In a 50-mL conical tube (Corning # 352070), 20 mL of each of the gelling agent and the 1 w/v% collagen solution was taken and uniformly mixed, and the resulting mixture was used as a collagen sol. The preparation of the collagen sol was performed under ice cooling. Subsequently, an acrylic cylindrical mold (Cosmos Vid, 38.8 mm (outer diameter) × 34.8 mm (inner diameter) × 30.0 mm (height)) was placed in a polystyrene rectangular dish (AS ONE Corporation # D210-16), and 10 mL of the collagen sol was filled in the mold. The dish was left to stand in a constant temperature machine at 37°C for 2 hours to cause fibril formation of collagen, whereby a collagen gel was obtained.
The collagen gel was vitrified by drying in a constant temperature and humidity machine (the temperature and humidity conditions: 10°C and 40% RH) for 2 days, whereby a collagen gel dried body was obtained. Further, the collagen gel dried body was hydrated by immersing it in D-PBS(-) in an amount of 1 mL per 10 mg of the collagen gel dried body, whereby a collagen vitrigel was obtained.
The collagen vitrigel was washed twice with D-PBS (-) to remove the excess salt, and the inside of the collagen vitrigel was equilibrated with D-PBS (-), and thereafter the collagen vitrigel was dried again in a constant temperature and humidity machine (the temperature and humidity conditions: 10°C and 40% RH) for 2 days, whereby a purified collagen vitrigel (dried body) was obtained.

Example 2

(Preparation of Gelling Agent)

3.21 g of sodium chloride (Wako Pure Chemical Industries, Ltd. # 191-01665), 3.40 g of potassium dihydrogen phosphate (Wako Pure Chemical Industries, Ltd. # 169-04245), and 1.85 g of sodium bicarbonate were taken and dissolved in 500 mL of sterile water, and the resulting solution was used as a gelling agent.

(Preparation of Collagen Vitrigel and Purified Collagen Vitrigel)

A collagen vitrigel and a purified collagen vitrigel (dried body) were prepared in the same manner as in Example 1 except that the gelling agent was changed to the above-prepared gelling agent.

Example 3

(Preparation of Gelling Agent)

3.21 g of sodium chloride and 1.85 g of sodium bicarbonate were taken and dissolved in 500 mL of sterile water, and the resulting solution was used as a gelling agent.

(Preparation of Collagen Vitrigel and Purified Collagen Vitrigel)

A collagen vitrigel and a purified collagen vitrigel (dried body) were prepared in the same manner as in Example 1 except that the gelling agent was changed to the above-prepared gelling agent.

Example 4

(Preparation of Gelling Agent)

To 500 mL of phosphate-buffered saline, 0.42 g of sodium bicarbonate was added and dissolved therein, whereby a gelling agent was prepared.

(Preparation of Collagen Vitrigel and Purified Collagen Vitrigel)

A collagen vitrigel and a purified collagen vitrigel (dried body) were prepared in the same manner as in Example 1 except that the gelling agent was changed to the above-prepared gelling agent.

Example 5

(Preparation of Gelling Agent)

To 500 mL of phosphate-buffered saline, 0.84 g of sodium bicarbonate was added and dissolved therein, whereby a gelling agent was prepared.

(Preparation of Collagen Vitrigel and Purified Collagen Vitrigel)

A collagen vitrigel and a purified collagen vitrigel (dried body) were prepared in the same manner as in Example 1 except that the gelling agent was changed to the above-prepared gelling agent.

Example 6

(Preparation of Gelling Agent)

To 500 mL of phosphate-buffered saline, 2.27 g of sodium bicarbonate was added and dissolved therein, whereby a gelling agent was prepared.

(Preparation of Collagen Vitrigel and Purified Collagen Vitrigel)

A collagen vitrigel and a purified collagen vitrigel (dried body) were prepared in the same manner as in Example 1 except that the gelling agent was changed to the above-prepared gelling agent.

Example 7

(Preparation of Gelling Agent)

To 500 mL of phosphate-buffered saline, 0.42 g of sodium bicarbonate and 0.53 g of sodium carbonate (Wako Pure Chemical Industries, Ltd. # 196-01595) were added and dissolved therein, whereby a gelling agent was prepared.

(Preparation of Collagen Vitrigel and Purified Collagen Vitrigel)

A collagen vitrigel and a purified collagen vitrigel (dried body) were prepared in the same manner as in Example 1 except that the gelling agent was changed to the above-prepared gelling agent.

Example 8

(Preparation of Gelling Agent)

To 500 mL of phosphate-buffered saline, 1.85 g of sodium bicarbonate and 0.53 g of sodium carbonate were added and dissolved therein, whereby a gelling agent was prepared.

(Preparation of Collagen Vitrigel and Purified Collagen Vitrigel)

A collagen vitrigel and a purified collagen vitrigel (dried body) were prepared in the same manner as in Example 1 except that the gelling agent was changed to the above-prepared gelling agent.

Example 9

(Preparation of Gelling Agent)

To 500 mL of phosphate-buffered saline, 2.94 g of sodium bicarbonate was added and dissolved therein, whereby a gelling agent was prepared.

(Preparation of Collagen Vitrigel and Purified Collagen Vitrigel)

A collagen vitrigel and a purified collagen vitrigel (dried body) were prepared in the same manner as in Example 1 except that the gelling agent was changed to the above-prepared gelling agent.

Example 10

(Preparation of Gelling Agent)

3.21 g of sodium chloride and 0.46 g of sodium bicarbonate were taken and dissolved in 500 mL of sterile water, and the resulting solution was used as a gelling agent.

(Preparation of Collagen Vitrigel and Purified Collagen Vitrigel)

A collagen vitrigel and a purified collagen vitrigel (dried body) were prepared in the same manner as in Example 1 except that the gelling agent was changed to the above-prepared gelling agent.

Example 11

(Preparation of Gelling Agent)

3.21 g of sodium chloride and 0.84 g of sodium bicarbonate were taken and dissolved in 500 mL of sterile water, and the resulting solution was used as a gelling agent.

(Preparation of Collagen Vitrigel and Purified Collagen Vitrigel)

A collagen vitrigel and a purified collagen vitrigel (dried body) were prepared in the same manner as in Example 1 except that the gelling agent was changed to the above-prepared gelling agent.

Example 12

(Preparation of Gelling Agent)

3.21 g of sodium chloride and 2.52 g of sodium bicarbonate were taken and dissolved in 500 mL of sterile water, and the resulting solution was used as a gelling agent.

(Preparation of Collagen Vitrigel and Purified Collagen Vitrigel)

A collagen vitrigel and a purified collagen vitrigel (dried body) were prepared in the same manner as in Example 1 except that the gelling agent was changed to the above-prepared gelling agent.

Example 13

(Preparation of Gelling Agent)

3.21 g of sodium chloride and 2.94 g of sodium bicarbonate were taken and dissolved in 500 mL of sterile water, and the resulting solution was used as a gelling agent.

(Preparation of Collagen Vitrigel and Purified Collagen Vitrigel)

A collagen vitrigel and a purified collagen vitrigel (dried body) were prepared in the same manner as in Example 1 except that the gelling agent was changed to the above-prepared gelling agent.

Example 14

(Preparation of Gelling Agent)

3.21 g of sodium chloride and 3.36 g of sodium bicarbonate were taken and dissolved in 500 mL of sterile water, and the resulting solution was used as a gelling agent.

(Preparation of Collagen Vitrigel and Purified Collagen Vitrigel)

A collagen vitrigel and a purified collagen vitrigel (dried body) were prepared in the same manner as in Example 1 except that the gelling agent was changed to the above-prepared gelling agent.

Example 15

(Preparation of Gelling Agent)

3.21 g of sodium chloride, 0.10 g of potassium chloride (Wako Pure Chemical Industries, Ltd. # 163-03545), 0.10 g of calcium chloride (Wako Pure Chemical Industries, Ltd. # 039-00475), 0.07 g of sodium dihydrogen phosphate monohydrate (Millipore # 10049-21-5), 0.42 g of sodium bicarbonate, and 0.53 g of sodium carbonate were taken and dissolved in 500 mL of sterile water, and the resulting solution was used as a gelling agent.

(Preparation of Collagen Vitrigel and Purified Collagen Vitrigel)

A collagen vitrigel and a purified collagen vitrigel (dried body) were prepared in the same manner as in Example 1 except that the gelling agent was changed to the above-prepared gelling agent.

Example 16

(Preparation of Gelling Agent)

3.21 g of sodium chloride, 0.10 g of potassium chloride, 0.10 g of calcium chloride, 0.07 g of sodium dihydrogen phosphate monohydrate, 1.85 g of sodium bicarbonate, and 1.59 g of sodium carbonate were taken and dissolved in 500 mL of sterile water, and the resulting solution was used as a gelling agent.

(Preparation of Collagen Vitrigel and Purified Collagen Vitrigel)

A collagen vitrigel and a purified collagen vitrigel (dried body) were prepared in the same manner as in Example 1 except that the gelling agent was changed to the above-prepared gelling agent.

Example 17

(Preparation of Gelling Agent)

1.17 g of sodium chloride and 4.62 g of sodium bicarbonate were taken and dissolved in 500 mL of sterile water, and the resulting solution was used as a gelling agent.

(Preparation of Collagen Vitrigel and Purified Collagen Vitrigel)

A collagen vitrigel and a purified collagen vitrigel (dried body) were prepared in the same manner as in Example 1 except that the gelling agent was changed to the above-prepared gelling agent.

Example 18

(Preparation of Gelling Agent)

3.21 g of sodium chloride, 0.10 g of potassium chloride, 0.10 g of calcium chloride, 0.07 g of sodium dihydrogen phosphate monohydrate, and 0.42 g of sodium bicarbonate were taken and dissolved in 500 mL of sterile water, and the resulting solution was used as a gelling agent.

(Preparation of Collagen Vitrigel and Purified Collagen Vitrigel)

A collagen vitrigel and a purified collagen vitrigel (dried body) were prepared in the same manner as in Example 1 except that the gelling agent was changed to the above-prepared gelling agent.

Example 19

(Preparation of Gelling Agent)

3.21 g of sodium chloride, 0.10 g of potassium chloride, 0.10 g of calcium chloride, 0.07 g of sodium dihydrogen phosphate monohydrate, and 0.84 g of sodium bicarbonate were taken and dissolved in 500 mL of sterile water, and the resulting solution was used as a gelling agent.

(Preparation of Collagen Vitrigel and Purified Collagen Vitrigel)

A collagen vitrigel and a purified collagen vitrigel (dried body) were prepared in the same manner as in Example 1 except that the gelling agent was changed to the above-prepared gelling agent.

Example 20

(Preparation of Gelling Agent)

3.21 g of sodium chloride, 0.10 g of potassium chloride, 0.10 g of calcium chloride, 0.07 g of sodium dihydrogen phosphate monohydrate, and 1.68 g of sodium bicarbonate were taken and dissolved in 500 mL of sterile water, and the resulting solution was used as a gelling agent.

(Preparation of Collagen Vitrigel and Purified Collagen Vitrigel)

A collagen vitrigel and a purified collagen vitrigel (dried body) were prepared in the same manner as in Example 1 except that the gelling agent was changed to the above-prepared gelling agent.

Example 21

(Preparation of Gelling Agent)

3.21 g of sodium chloride, 0.10 g of potassium chloride, 0.10 g of calcium chloride, 0.07 g of sodium dihydrogen phosphate monohydrate, and 2.52 g of sodium bicarbonate were taken and dissolved in 500 mL of sterile water, and the resulting solution was used as a gelling agent.

(Preparation of Collagen Vitrigel and Purified Collagen Vitrigel)

A collagen vitrigel and a purified collagen vitrigel (dried body) were prepared in the same manner as in Example 1 except that the gelling agent was changed to the above-prepared gelling agent.

Example 22

(Preparation of Gelling Agent)

3.21 g of sodium chloride, 0.10 g of potassium chloride, 0.10 g of calcium chloride, 0.07 g of sodium dihydrogen phosphate monohydrate, and 3.36 g of sodium bicarbonate were taken and dissolved in 500 mL of sterile water, and the resulting solution was used as a gelling agent.

(Preparation of Collagen Vitrigel and Purified Collagen Vitrigel)

A collagen vitrigel and a purified collagen vitrigel (dried body) were prepared in the same manner as in Example 1 except that the gelling agent was changed to the above-prepared gelling agent.

Example 23

(Preparation of Gelling Agent)

3.21 g of sodium chloride, 0.10 g of potassium chloride, 0.10 g of calcium chloride, 0.07 g of sodium dihydrogen phosphate monohydrate, and 4.20 g of sodium bicarbonate were taken and dissolved in 500 mL of sterile water, and the resulting solution was used as a gelling agent.

(Preparation of Collagen Vitrigel and Purified Collagen Vitrigel)

A collagen vitrigel and a purified collagen vitrigel (dried body) were prepared in the same manner as in Example 1 except that the gelling agent was changed to the above-prepared gelling agent.

Example 24

(Preparation of Gelling Agent)

3.21 g of sodium chloride, 0.10 g of potassium chloride, 0.10 g of calcium chloride, 0.07 g of sodium dihydrogen phosphate monohydrate, and 0.53 g of sodium carbonate were taken and dissolved in 500 mL of sterile water, and the resulting solution was used as a gelling agent.

(Preparation of Collagen Vitrigel and Purified Collagen Vitrigel)

A collagen vitrigel and a purified collagen vitrigel (dried body) were prepared in the same manner as in Example 1 except that the gelling agent was changed to the above-prepared gelling agent.

Example 25

(Preparation of Gelling Agent)

3.21 g of sodium chloride, 0.10 g of potassium chloride, 0.10 g of calcium chloride, 0.07 g of sodium dihydrogen phosphate monohydrate, and 1.06 g of sodium carbonate were taken and dissolved in 500 mL of sterile water, and the resulting solution was used as a gelling agent.

(Preparation of Collagen Vitrigel and Purified Collagen Vitrigel)

A collagen vitrigel and a purified collagen vitrigel (dried body) were prepared in the same manner as in Example 1 except that the gelling agent was changed to the above-prepared gelling agent.

Example 26

(Preparation of Gelling Agent)

3.21 g of sodium chloride, 0.10 g of potassium chloride, 0.10 g of calcium chloride, 0.07 g of sodium dihydrogen phosphate monohydrate, 0.84 g of sodium bicarbonate, and 0.27 g of sodium carbonate were taken and dissolved in 500 mL of sterile water, and the resulting solution was used as a gelling agent.

(Preparation of Collagen Vitrigel and Purified Collagen Vitrigel)

A collagen vitrigel and a purified collagen vitrigel (dried body) were prepared in the same manner as in Example 1 except that the gelling agent was changed to the above-prepared gelling agent.

Example 27

(Preparation of Gelling Agent)

3.21 g of sodium chloride, 0.10 g of potassium chloride, 0.10 g of calcium chloride, 0.07 g of sodium dihydrogen phosphate monohydrate, 0.84 g of sodium bicarbonate, and 0.53 g of sodium carbonate were taken and dissolved in 500 mL of sterile water, and the resulting solution was used as a gelling agent.

(Preparation of Collagen Vitrigel and Purified Collagen Vitrigel)

A collagen vitrigel and a purified collagen vitrigel (dried body) were prepared in the same manner as in Example 1 except that the gelling agent was changed to the above-prepared gelling agent.

Example 28

(Preparation of Gelling Agent)

3.21 g of sodium chloride, 0.10 g of potassium chloride, 0.10 g of calcium chloride, 0.07 g of sodium dihydrogen phosphate monohydrate, 0.84 g of sodium bicarbonate, and 1.06 g of sodium carbonate were taken and dissolved in 500 mL of sterile water, and the resulting solution was used as a gelling agent.

(Preparation of Collagen Vitrigel and Purified Collagen Vitrigel)

A collagen vitrigel and a purified collagen vitrigel (dried body) were prepared in the same manner as in Example 1 except that the gelling agent was changed to the above-prepared gelling agent.

Example 29

(Preparation of Gelling Agent)

To 500 mL of phosphate-buffered saline, 0.21 g of sodium bicarbonate was added and dissolved therein, whereby a gelling agent was prepared.

(Preparation of Collagen Vitrigel and Purified Collagen Vitrigel)

A collagen vitrigel and a purified collagen vitrigel (dried body) were prepared in the same manner as in Example 1 except that the gelling agent was changed to the above-prepared gelling agent.

Example 30

(Preparation of Gelling Agent)

To 500 mL of a Hanks' Balanced salt solution (HBSS(-), Wako Pure Chemical Industries, Ltd. # 085-09355), 0.85 g of sodium carbonate was added and dissolved therein, whereby a gelling agent was prepared.

(Preparation of Collagen Vitrigel and Purified Collagen Vitrigel)

A collagen vitrigel and a purified collagen vitrigel (dried body) were prepared in the same manner as in Example 1 except that the gelling agent was changed to the above-prepared gelling agent.

Example 31

(Preparation of Gelling Agent)

3.21 g of sodium chloride, 0.10 g of potassium chloride, 0.10 g of calcium chloride, 0.07 g of sodium dihydrogen phosphate monohydrate, and 0.27 g of sodium carbonate were taken and dissolved in 500 mL of sterile water, and the resulting solution was used as a gelling agent.

(Preparation of Collagen Vitrigel and Purified Collagen Vitrigel)

A collagen vitrigel and a purified collagen vitrigel (dried body) were prepared in the same manner as in Example 1 except that the gelling agent was changed to the above-prepared gelling agent.

Comparative Example 1

(Preparation of Gelling Agent)

To 490 mL of Dulbecco's modified Eagle's medium (DMEM), 10 mL of 1 mol/L HEPES (4-(2-hydroxyethyl)-1-piperazine ethanesulfonic acid), Thermo Fisher Scientific # 15630-080) was added and uniformly mixed therein, and the resulting mixture was used as a gelling agent.

(Preparation of Collagen Vitrigel and Collagen Vitrigel Dried body)

In a 50-mL conical tube, 20 mL each of the above-prepared gelling agent and the 1 w/v% collagen solution of Example 1 was taken and uniformly mixed, and the resulting mixture was used as a collagen sol. The preparation of the collagen sol was performed under ice cooling. Subsequently, an acrylic cylindrical mold was placed in a polystyrene rectangular dish, and 10 mL of the collagen sol was filled in the mold. The dish was left to stand in a carbon dioxide gas incubator ($CO_2$ 5%) at 37°C for 2 hours to cause fibril formation of collagen, whereby a collagen gel was obtained.
The collagen gel was dried and vitrified in a constant temperature and humidity machine, whereby a collagen gel dried body was obtained. Further, the collagen gel dried body was hydrated by immersing it in D-PBS (-) in an amount of 1 mL per 10 mg of the collagen gel dried body, whereby a collagen vitrigel was obtained. The collagen vitrigel was washed twice with D-PBS(-) to remove the excess salt and other components, and the inside of the collagen vitrigel was equilibrated with D-PBS(-), followed by drying again, whereby a corresponding dried body of the collagen vitrigel was obtained.

Comparative Example 2

(Preparation of Collagen Vitrigel)

Preparation of a collagen vitrigel was attempted by the method of Example 1 except that the gelling agent was changed to D-PBS(-). However, the once gelled collagen gel was dissolved and transformed into a sol in the subsequent step, and therefore, the material could not be recovered as a collagen gel dried body, and a collagen vitrigel could not be obtained.

Comparative Example 3

(Preparation of Gelling Agent)

8.77 g of sodium chloride was taken and dissolved in 500 mL of sterile water, and the resulting solution was used as a gelling agent.

(Preparation of Collagen Vitrigel)

Preparation of a collagen vitrigel was attempted by the method of Example 1 except that the gelling agent was changed to the above-prepared gelling agent. However, the once gelled collagen gel was dissolved and transformed into a sol in the subsequent step, and therefore, the material could not be recovered as a collagen gel dried body, and a collagen vitrigel could not be obtained.

Comparative Example 4

(Preparation of Gelling Agent)

1.75 g of sodium chloride, 0.14 g of sodium dihydrogen phosphate monohydrate, and 1.35 g of disodium hydrogen phosphate (Wako Pure Chemical Industries, Ltd. # 197-02865) were taken and dissolved in 500 mL of sterile water, and the resulting solution was used as a gelling agent.

(Preparation of Collagen Vitrigel)

Preparation of a collagen vitrigel was attempted by the method of Example 1 except that the gelling agent was changed to the above-prepared gelling agent. However, the once gelled collagen gel was dissolved and transformed into a sol in the subsequent step, and therefore, the material could not be recovered as a collagen gel dried body, and a collagen vitrigel could not be obtained.

Comparative Example 5

(Preparation of Collagen Vitrigel)

Preparation of a collagen vitrigel was attempted by the method of Example 1 except that the gelling agent was changed to HBSS(-). However, collagen was not gelled, and therefore, a collagen gel could not be obtained, so that a collagen vitrigel could not be obtained.

Comparative Example 6

(Preparation of Gelling Agent)

To 500 mL of HBSS(-), 2.12 g of sodium carbonate was added and dissolved therein, whereby a gelling agent was prepared.

(Preparation of Collagen Vitrigel)

Preparation of a collagen vitrigel was attempted by the method of Example 1 except that the gelling agent was changed to the above-prepared gelling agent. However, collagen was not gelled, and therefore, a collagen gel could not be obtained, so that a collagen vitrigel could not be obtained.

Comparative Example 7

(Preparation of Gelling Agent)

To 500 mL of HBSS(-), 4.24 g of sodium carbonate was added and dissolved therein, whereby a gelling agent was prepared.

(Preparation of Collagen Vitrigel)

Preparation of a collagen vitrigel was attempted by the method of Example 1 except that the gelling agent was changed to the above-prepared gelling agent. However, collagen was not gelled, and therefore, a collagen gel could not be obtained, so that a collagen vitrigel could not be obtained.

Comparative Example 8

(Preparation of Gelling Agent)

3.21 g of sodium chloride, 0.10 g of potassium chloride, 0.10 g of calcium chloride, and 0.07 g of sodium dihydrogen phosphate monohydrate were taken and dissolved in 500 mL of sterile water, and the resulting solution was used as a gelling agent.

(Preparation of Collagen Vitrigel)

Preparation of a collagen vitrigel was attempted by the method of Example 1 except that the gelling agent was changed to the above-prepared gelling agent. However, collagen was not gelled, and therefore, a collagen gel could not be obtained, so that a collagen vitrigel could not be obtained.

Comparative Example 9

(Preparation of Gelling Agent)

3.21 g of sodium chloride, 0.10 g of potassium chloride, 0.10 g of calcium chloride, 0.07 g of sodium dihydrogen phosphate monohydrate, and 0.21 g of sodium bicarbonate were taken and dissolved in 500 mL of sterile water, and the resulting solution was used as a gelling agent.

(Preparation of Collagen Vitrigel)

Preparation of a collagen vitrigel was attempted by the method of Example 1 except that the gelling agent was changed to the above-prepared gelling agent. However, collagen was not gelled, and therefore, a collagen gel could not be obtained, so that a collagen vitrigel could not be obtained.

Comparative Example 10

(Preparation of Gelling Agent)

3.21 g of sodium chloride, 0.10 g of potassium chloride, 0.10 g of calcium chloride, 0.07 g of sodium dihydrogen phosphate monohydrate, and 2.12 g of sodium carbonate were taken and dissolved in 500 mL of sterile water, and the resulting solution was used as a gelling agent.

(Preparation of Collagen Vitrigel)

Preparation of a collagen vitrigel was attempted by the method of Example 1 except that the gelling agent was changed to the above-prepared gelling agent. However, collagen was not gelled, and therefore, a collagen gel could not be obtained, so that a collagen vitrigel could not be obtained.

Test Example 1

Measurement of Physical Properties:

The concentrations of the inorganic compounds contained in the collagen sols of Examples 1 to 31 and Comparative Examples 1 to 10, and the ionic strengths and pH thereof, and the film strengths of the purified collagen vitrigels (hydrated bodies) of Examples 1 to 3, Example 8, Example 10, Example 12, Example 18, Examples 23 to 25, Example 27, Examples 29 to 31, and Comparative Example 1 were measured by the following methods. The results are shown in Table 1 to Table 7.

(pH Measurement)

The pH was measured for the collagen sols before gelling of Examples 1 to 29 and Comparative Examples 1 to 10. The pH measurement was performed by placing 0.5 mL of each collagen sol in a measurement electrode dish of a pH meter (HORIBA, Ltd. # LAQUAtwin AS-712) and measuring the pH.

(Calculation of Ionic Strength)

When the molar concentrations (mol/L) of ion species A, B, C, ... of the compounds (except for collagen) contained in a collagen sol are represented by $m_A$, $m_B$, $m_C$, ..., respectively, and the charge numbers thereof are represented by $Z_A$, $Z_B$, $Z_C$, ..., respectively, the ionic strength I of the collagen sol is defined by the following formula.

Formula 1

$$I = \frac{1}{2}\sum_i m_i Z_i^2 \quad (i = A 、 B 、 C . . . )$$

(Measurement of Film Strength)

The film strengths of the purified collagen vitrigels (hydrated bodies) of Examples 1 to 3, Example 8, Example 10, Example 12, Example 18, Examples 23 to 25, Example 27, Examples 29 to 31, and Comparative Example 1 were measured. The film strengths of the purified collagen vitrigels (hydrated bodies) were measured by the following method. That is, the purified collagen vitrigel (dried body) having a diameter of 34.8 mm was rehydrated by immersing it in 8 mL of D-PBS(-) for 1 hour. The purified collagen vitrigel (hydrated body) obtained by hydration was fixed by being sandwiched between two acrylic plates with a diameter of 50 mm having a hole with a diameter of 5 mm at the center. The fixed film exposed from the hole with a diameter of 5 mm was vertically pierced with a needle made of stainless steel with a diameter of 1.0 mm at a rate of 3.0 mm/min, and the maximum stress (N) before the needle penetrated through the purified collagen vitrigel (hydrated body) was measured using a small desktop testing machine (Shimadzu Corporation # EZ-SX, load cell maximum load 5 N) . The measurement was performed at three sites per purified collagen vitrigel (hydrated body), and the average was taken as the maximum stress (N). The operation was performed for three purified collagen vitrigels (hydrated bodies), and the average of the three maximum stresses was taken as the membrane strength (N).

[Table 1]

| | | Example 1 | Example 2 | Example 3 | Example 4 | Example 5 | Example 6 |
|---|---|---|---|---|---|---|---|
| Concentration (mmol/L) | NaCl | 68 | 55 | 55 | 68 | 68 | 68 |
| | KCl | 1.4 | - | - | 1.4 | 1.4 | 1.4 |
| | $CaCl_2$ | - | - | - | - | - | - |
| | $MgSO_4$ | - | - | - | - | - | - |
| | $NaH_2PO_4$ | - | - | - | - | - | - |
| | $KH_2PO_4$ | 0.8 | 5 | - | 0.8 | 0.8 | 0.8 |
| | $Na_2HPO_4$ | 4 | - | - | 4 | 4 | 4 |
| | $NaHCO_3$ | 22 | 22 | 22 | 5 | 10 | 27 |
| | $Na_2CO_3$ | - | - | - | - | - | - |
| Components other than inorganic salts | | Absence | Absence | Absence | Absence | Absence | Absence |
| Ionic strength | | 0.165 | 0.151 | 0.121 | 0.113 | 0.128 | 0.179 |
| pH | | 7.5 | 7.3 | 7.4 | 7.2 | 7.2 | 7.4 |
| Film strength (N) (average $\pm$ standard deviation) | | 0.021 $\pm$ 0.002 | 0.025 $\pm$ 0.002 | 0.028 $\pm$ 0.003 | No data | No data | No data |
| Remarks | | | | | | | |

[Table 2]

| | | Example 7 | Example 8 | Example 9 | Example 10 | Example 11 | Example 12 |
|---|---|---|---|---|---|---|---|
| Concentration (mmol/L) | NaCl | 68 | 68 | 68 | 55 | 55 | 55 |
| | KCl | 1.4 | 1.4 | 1.4 | - | - | - |
| | $CaCl_2$ | - | - | - | - | - | - |
| | $MgSO_4$ | | | | | | |
| | $NaH_2PO_4$ | - | - | - | - | - | - |
| | $KH_2PO_4$ | 0.8 | 0.8 | 0.8 | - | - | - |
| | $Na_2HPO_4$ | 4 | 4 | 4 | - | - | - |
| | $NaHCO_3$ | 5 | 22 | 35 | 5.5 | 10 | 30 |
| | $Na_2CO_3$ | 5 | 5 | - | - | - | - |
| Components other than inorganic salts | | Absence | Absence | Absence | Absence | Absence | Absence |
| Ionic strength | | 0.128 | 0.179 | 0.203 | 0.072 | 0.085 | 0.145 |
| pH | | 9.0 | 8.6 | 7.7 | 7.0 | 7.0 | 7.4 |
| Film strength (N) (average ± standard deviation) | | No data | 0.067 ± 0.013 | No data | 0.024 ± 0.003 | No data | 0.025 ± 0.004 |
| Remarks | | | | | | | |

[Table 3]

| | | Example 13 | Example 14 | Example 15 | Example 16 | Example 17 | Example 18 |
|---|---|---|---|---|---|---|---|
| Concentration (mmol/L) | NaCl | 55 | 55 | 55 | 55 | 20 | 55 |
| | KCl | - | - | 2.6 | 2.6 | - | 2.6 |
| | $CaCl_2$ | - | - | 0.9 | 0.9 | - | 0.9 |
| | $MgSO_4$ | - | - | - | - | - | - |
| | $NaH_2PO_4$ | - | - | 0.5 | 0.5 | - | 0.5 |
| | $KH_2PO_4$ | - | - | - | - | - | - |
| | $Na_2HPO_4$ | - | - | - | - | - | - |
| | $NaHCO_3$ | 35 | 40 | 5 | 22 | 55 | 5 |
| | $Na_2CO_3$ | - | - | 5 | 15 | - | - |
| Components other than inorganic salts | | Absence | Absence | Absence | Absence | Absence | Absence |
| Ionic strength | | 0.160 | 0.175 | 0.093 | 0.174 | 0.185 | 0.078 |
| pH | | 7.5 | 7.7 | 9.0 | 9.4 | 7.9 | 6.3 |
| Film strength (N) (average ± standard deviation) | | No data | No data | No data | No data | No data | 0.019±0.002 |
| Remarks | | | | | | | |

[Table 4]

| | | Example 19 | Example 20 | Example 21 | Example 22 | Example 23 | Example 24 |
|---|---|---|---|---|---|---|---|
| Concentration (mmol/L) | NaCl | 55 | 55 | 55 | 55 | 55 | 55 |
| | KCl | 2.6 | 2.6 | 2.6 | 2.6 | 2.6 | 2.6 |
| | $CaCl_2$ | 0.9 | 0.9 | 0.9 | 0.9 | 0.9 | 0.9 |
| | $MgSO_4$ | - | - | - | - | - | - |
| | $NaH_2PO_4$ | 0.5 | 0.5 | 0.5 | 0.5 | 0.5 | 0.5 |
| | $KH_2PO_4$ | - | - | - | - | - | - |
| | $Na_2HPO_4$ | - | - | - | - | - | - |
| | $NaHCO_3$ | 10 | 20 | 30 | 40 | 50 | - |
| | $Na_2CO_3$ | - | - | - | - | - | 5 |
| Components other than inorganic salts | | Absence | Absence | Absence | Absence | Absence | Absence |
| Ionic strength | | 0.093 | 0.123 | 0.153 | 0.183 | 0.213 | 0.078 |
| pH | | 6.7 | 7.1 | 7.2 | 7.8 | 8.5 | 9.3 |
| Film strength (N) (average ± standard deviation) | | No data | No data | No data | No data | 0.033 ± 0.005 | 0.042 ± 0.005 |
| Remarks | | | | | | | |

[Table 5]

| | | Example 25 | Example 26 | Example 27 | Example 28 | Example 29 | Example 30 |
|---|---|---|---|---|---|---|---|
| Concentration (mmol/L) | NaCl | 55 | 55 | 55 | 55 | 68 | 68.5 |
| | KCl | 2.6 | 2.6 | 2.6 | 2.6 | 1.4 | 2.7 |
| | $CaCl_2$ | 0.9 | 0.9 | 0.9 | 0.9 | - | - |
| | $MgSO_4$ | - | - | - | - | | |
| | $NaH_2PO_4$ | 0.5 | 0.5 | 0.5 | 0.5 | - | - |
| | $KH_2PO_4$ | - | - | - | - | 0.8 | 0.2 |
| | $Na_2HPO_4$ | - | - | - | - | 4 | 0.2 |
| | $NaHCO_3$ | - | 10 | 10 | 10 | 2.5 | 2.1 |
| | $Na_2CO_3$ | 10 | 2.5 | 5 | 10 | - | 8.0 |
| Components other than inorganic salts | | Absence | Absence | Absence | Absence | Absence | Absence |
| Ionic strength | | 0.093 | 0.101 | 0.108 | 0.123 | 0.106 | 0.104 |
| pH | | 9.8 | 8.1 | 8.6 | 9.3 | 6.6 | 9.6 |
| Film strength (N) (average ± standard deviation) | | 0.053 ± 0.008 | No data | 0.053 ± 0.005 | No data | 0.011 ± 0.002 | 0.054 ± 0.005 |
| Remarks | | | | | | | |

[Table 6]

| | | Example 31 | Comparative Example 1 | Comparative Example 2 | Comparative Example 3 | Comparative Example 4 | Comparative Example 5 |
|---|---|---|---|---|---|---|---|
| Concentration (mmol/L) | NaCl | 55 | 55 | 68 | 150 | 30 | 68.5 |
| | KCl | 2.6 | 2.6 | 1.4 | - | - | 2.7 |
| | CaCl$_2$ | 0.9 | 0.9 | - | - | - | - |
| | MgSO$_4$ | - | 0.4 | | | | |
| | NaH$_2$PO$_4$ | 0.5 | 0.5 | - | - | 1 | - |
| | KH$_2$PO$_4$ | - | - | 0.8 | - | - | 0.2 |
| | Na$_2$HPO$_4$ | - | - | 4 | - | 19 | 0.2 |
| | NaHCO$_3$ | - | 22 | - | - | - | 2.1 |
| | Na$_2$CO$_3$ | 2.5 | - | - | - | - | - |
| Components other than inorganic salts | | Absence | Presence (a total of 27 components such as amino acids, vitamins, sugars, HEPES, etc.) | Absence | Absence | Absence | Absence |
| Ionic strength | | 0.071 | 0.131 | 0.098 | 0.150 | 0.150 | 0.080 |
| pH | | 6.7 | 7.0 | 5.1 | 3.1 | 7.4 | 4.3 |
| Film strength (N) (average ± standard deviation) | | 0.024 ± 0.002 | 0.022 ± 0.001 | - | - | - | - |
| Remarks | | | | Could not be produced | Could not be produced | Could not be produced | Could not be produced |

[Table 7]

| | | Comparative Example 6 | Comparative Example 7 | Comparative Example 8 | Comparative Example 9 | Comparative Example 10 |
|---|---|---|---|---|---|---|
| Concentration (mmol/L) | NaCl | 68.5 | 68.5 | 55 | 55 | 55 |
| | KCl | 2.7 | 2.7 | 2.6 | 2.6 | 2.6 |
| | $CaCl_2$ | - | - | 0.9 | 0.9 | 0.9 |
| | $MgSO_4$ | | | - | - | - |
| | $NaH_2PO_4$ | - | - | 0.5 | 0.5 | 0.5 |
| | $KH_2PO_4$ | 0.2 | 0.2 | - | - | - |
| | $Na_2HPO_4$ | 0.2 | 0.2 | - | - | - |
| | $NaHCO_3$ | 2.1 | 2.1 | - | 2.5 | - |
| | $Na_2CO_3$ | 20 | 40 | - | - | 20 |
| Components other than inorganic salts | | Absence | Absence | Absence | Absence | Absence |
| Ionic strength | | 0.140 | 0.200 | 0.063 | 0.071 | 0.123 |
| pH | | 10.0 | 10.5 | 3.2 | 4.4 | 10.2 |
| Film strength (N) (average $\pm$ standard deviation) | | | | | | |
| Remarks | | Could not be produced | Could not be produced | Could not be produced | Could not be produced | Could not be produced |

As apparent from Table 1 to Table 6, the purified collagen vitrigels obtained according to the method of the present invention had a sufficient film strength, and also did not substantially contain residual components other than the inorganic salts, were biologically highly safe, and were of practical use.

Industrial Applicability

As described above, according to the production methods of the present invention, a collagen vitrigel and a purified product thereof that are biologically highly safe are obtained easily at low cost.

Moreover, the obtained collagen vitrigel and purified collagen vitrigel have a sufficient film strength, are easily produced and processed industrially and mechanically, are easy to handle, and are highly safe, and therefore, are useful as various biological graft materials, for example, a cell carrier for regenerative medicine, a wound dressing, an artificial skin, an adhesion preventive material, and the like.

**Claims**

1. A method for producing a collagen vitrigel, **characterized by** gelling collagen with a gelling agent containing an inorganic carbonate and a compound selected from the group consisting of an inorganic chloride and an inorganic phosphate, subsequently vitrifying the obtained collagen gel, and further subjecting the vitrified collagen gel to a hydration treatment.

2. The method for producing a collagen vitrigel according to claim 1, wherein the collagen is porcine skin-derived atelocollagen.

3. The method for producing a collagen vitrigel according to claim 1 or 2, wherein the pH at 25°C of a mixture of the collagen and the gelling agent is 6.2 to 9.8.

4. The method for producing a collagen vitrigel according to any one of claims 1 to 3, wherein the ionic strength of a compound contained in the mixture of the collagen and the gelling agent is 0.07 to 0.22.

5. The method for producing a collagen vitrigel according to any one of claims 1 to 4, wherein the inorganic carbonate is an inorganic carbonate or an inorganic bicarbonate.

6. The method for producing a collagen vitrigel according to claim 5, wherein the inorganic carbonate is sodium carbonate or potassium carbonate.

7. The method for producing a collagen vitrigel according to claim 5 or 6, wherein the inorganic bicarbonate is sodium bicarbonate or potassium bicarbonate.

8. The method for producing a collagen vitrigel according to any one of claims 1 to 7, wherein the inorganic chloride is one or more types selected from the group consisting of sodium chloride, potassium chloride, magnesium chloride, and calcium chloride.

9. The method for producing a collagen vitrigel according to any one of claims 1 to 8, wherein the inorganic phosphate is one or more types selected from the group consisting of sodium phosphate, potassium phosphate, disodium hydrogen phosphate, dipotassium hydrogen phosphate, sodium dihydrogen phosphate, potassium dihydrogen phosphate, and calcium dihydrogen phosphate.

10. The method for producing a collagen vitrigel according to any one of claims 1 to 9, wherein the gelling agent does not contain an organic component derived from a medium component and an organic buffer component.

11. A method for producing a purified collagen vitrigel hydrated body, **characterized by** desalting and equilibrating a collagen vitrigel prepared by the production method according to any one of claims 1 to 10.

12. A method for producing a purified collagen vitrigel dried body, **characterized by** further drying and revitrifying a purified collagen vitrigel hydrated body prepared by the production method according to claim 11.

13. A method for producing a purified collagen vitrigel hydrated body, **characterized by** further rehydrating a purified collagen vitrigel dried body prepared by the production method according to claim 12.

14. A collagen vitrigel obtained by the method according to any one of claims 1 to 10.

15. A biological graft material, comprising at least the collagen vitrigel according to claim 14.

16. The biological graft material according to claim 15, wherein cells are not carried before use.

17. A medical artificial skin, comprising at least the collagen vitrigel according to claim 14.

18. A purified collagen vitrigel hydrated body obtained by the method according to either claim 11 or claim 13.

19. A biological graft material, comprising at least the purified collagen vitrigel hydrated material according to claim 18.

20. The biological graft material according to claim 19, wherein cells are not carried before use.

21. A medical artificial skin, comprising at least the purified collagen vitrigel hydrated material according to claim 18.

22. A purified collagen vitrigel dried body obtained by the method according to claim 12.

23. A biological graft material, comprising at least the purified collagen vitrigel dried body according to claim 22.

24. The biological graft material according to claim 23, wherein cells are not carried before use.

25. A medical artificial skin, comprising at least the purified collagen vitrigel dried body according to claim 22.

26. A collagen gel dried body obtained by gelling collagen with a gelling agent containing an inorganic carbonate and a compound selected from the group consisting of an inorganic chloride and an inorganic phosphate, subsequently vitrifying the obtained collagen gel.

[FIG.1]

```
            ┌─────────────────────────────┐
            │        Collagen sol         │
            └─────────────────────────────┘
                    ↓ Gelation
            ┌─────────────────────────────┐
            │        Collagen gel         │
            └─────────────────────────────┘
                    ↓ Vitrification (drying)
            ┌─────────────────────────────┐
            │     Dried collagen gel      │
            └─────────────────────────────┘
                    ↓ Rehydration
            ┌─────────────────────────────┐
            │      Collagen vitrigel      │
            └─────────────────────────────┘
                    ↓ Washing (desalting, equilibration)
    ┌─────────────────────────────────────────────┐
    │  Purified collagen vitrigel (hydrated body) │
    └─────────────────────────────────────────────┘
         ↑ Rehydration     ↓ Re-vitrification (re-drying)
    ┌─────────────────────────────────────────────┐
    │    Purified collagen vitigel (dried body)   │
    └─────────────────────────────────────────────┘
```

## INTERNATIONAL SEARCH REPORT

| International application No. |
| --- |
| PCT/JP2018/025846 |

### A. CLASSIFICATION OF SUBJECT MATTER
Int. Cl. C07K14/78(2006.01)i, A61L27/02(2006.01)i, A61L27/24(2006.01)i, A61L27/60(2006.01)i

According to International Patent Classification (IPC) or to both national classification and IPC

### B. FIELDS SEARCHED
Minimum documentation searched (classification system followed by classification symbols)
Int. Cl. C07K14/78, A61L27/02, A61L27/24, A61L27/60

Documentation searched other than minimum documentation to the extent that such documents are included in the fields searched
Published examined utility model applications of Japan     1922-1996
Published unexamined utility model applications of Japan   1971-2018
Registered utility model specifications of Japan           1996-2018
Published registered utility model applications of Japan   1994-2018

Electronic data base consulted during the international search (name of data base and, where practicable, search terms used)
JSTPlus/JMEDPlus/JST7580 (JDreamIII), CAplus/MEDLINE/EMBASE/BIOSIS (STN), WPIDS (STN)

### C. DOCUMENTS CONSIDERED TO BE RELEVANT

| Category* | Citation of document, with indication, where appropriate, of the relevant passages | Relevant to claim No. |
| --- | --- | --- |
| Y | 押方歩他，コラーゲンビトリゲル新素材の開発，医薬品作物,医療用素材等の開発 研究成果，vol. 561, 2016, pp. 315-322, entire text, p. 315, left column, p. 315, right column to p. 321, p. 321, left column, non-official translation (OSHIKATA, Ayumi. Development of new material from collagen-vitrigel. Research for agri-health translational research project. Research Findings) | 1-26 |
| Y | 許南浩編，よく使われる培地(DMEM,ハム F12 など)はどうやって開発されたの?，細胞培養なるほど Q&A，第 3 刷，株式会社羊土社，2005, Chapter 4, pp. 82-83, Table 1, non-official translation (HOH, Nanho ed. How Are Commonly-Used Culture Media (DMEM, Ham F12, etc.) Developed? Cell Culture Q&A, 3rd Printing, Yodosha Co., Ltd.) | 1-26 |

☒ Further documents are listed in the continuation of Box C.   ☐ See patent family annex.

| | | |
| --- | --- | --- |
| * | Special categories of cited documents: | |
| "A" | document defining the general state of the art which is not considered to be of particular relevance | |
| "E" | earlier application or patent but published on or after the international filing date | |
| "L" | document which may throw doubts on priority claim(s) or which is cited to establish the publication date of another citation or other special reason (as specified) | |
| "O" | document referring to an oral disclosure, use, exhibition or other means | |
| "P" | document published prior to the international filing date but later than the priority date claimed | |

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
| --- | --- |
| 26.09.2018 | 09.10.2018 |

| Name and mailing address of the ISA/ | Authorized officer |
| --- | --- |
| Japan Patent Office<br>3-4-3, Kasumigaseki, Chiyoda-ku,<br>Tokyo 100-8915, Japan | |
| | Telephone No. |

Form PCT/ISA/210 (second sheet) (January 2015)

**INTERNATIONAL SEARCH REPORT**

| International application No. |
|---|
| PCT/JP2018/025846 |

C (Continuation).    DOCUMENTS CONSIDERED TO BE RELEVANT

| Category* | Citation of document, with indication, where appropriate, of the relevant passages | Relevant to claim No. |
|---|---|---|
| A | WO 2016/068228 A1 (KOKEN CO., LTD.) 06 May 2016, entire text (Family: none) | 1-26 |
| A | JP 2017-86066 A (TAKI CHEMICAL CO., LTD.) 25 May 2017, entire text (Family: none) | 1-26 |
| A | WO 2012/026531 A1 (NATIONAL INSTITUTE OF AGROBIOLOGICAL SCIENCES) 01 March 2012, entire text & US 2013/0217126 A1 & US 2014/0319728 A1 & EP 2610335 A1 | 1-26 |

Form PCT/ISA/210 (continuation of second sheet) (January 2015)

# EP 3 689 901 A1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6071468 B

- JP 4674211 B

**Non-patent literature cited in the description**

- **KATSURA SUGAWARA.** Recent Advancement in the Treatment of Cartilage Defects by Cultured Cartilage. *Journal of Artificial Organs, The Official Journal of the Japanese Society for Artificial Organs,* 2013, vol. 42 (3), 198-200
- **TOSHIAKI TAKEZAWA.** Collagen Vitrigel: A Novel Scaffold That Can Facilitate a Three-Dimensional Caluture for Reconstructing Organoids. *Cell Transplantaion,* 2004, vol. 13, 463-473

- **HIDEAKI MARUKI.** Effects of a cell-free method using collagen vitrigel incorporating TGF-$\beta$1 on articular cartilage repair in a rabbit osteochondral defect model. *Journal of Biomedical Materials Research B,* 2016
- Ministry of Agriculture, Forestry and Fisheries Agriculture, Forestry and Fisheries Research Council Secretariat. *Research for agri-health translational research project, Research Findings,* 2016, vol. 561, 315-322
- **TAKUSHI EISEI.** Edible eyeballs from fish. *Nature,* 1990, vol. 345, 298-299

27